# EUROPÄISCHE PATENTSCHRIFT

(11) **EP 1 390 154 B1**
(45) Veröffentlichungstag und Bekanntmachung des Hinweises auf die Patenterteilung: **10.11.2004**
(21) Anmeldenummer: 02740288.2
(22) Anmeldetag: 24.04.2002
(51) Int. Cl.: B05B 11/00, A61M 15/00

(54) **VORRICHTUNG ZUM VERSPRÜHEN VON FLÜSSIGKEITEN**
DEVICE FOR THE SPRAYING OF FLUIDS
PULVERISATEUR DE LIQUIDES

(30) Priorität: 30.04.2001 DE 10121232
(43) Veröffentlichungstag der Anmeldung: 25.02.2004
(73) Patentinhaber: E. Braun GmbH, 79353 Bahlingen (DE)
(72) Erfinder: BELLER, Klaus-Dieter, 79341 Kenzingen (DE)
(74) Vertreter: Goy, Wolfgang, Dipl.-Phys.
(86) Internationale Anmeldenummer: PCT/DE2002/001492
(87) Internationale Veröffentlichungsnummer: WO 2002/087777

(56) Entgegenhaltungen:
- WO-A-95/00195
- US-A- 4 361 253
- US-A- 4 526 302
- US-A- 5 595 326
- US-A- 5 927 559

## Beschreibung

Die Erfindung betrifft eine Vorrichtung zum Versprühen von Flüssigkeiten nach dem Oberbegriff des Anspruchs 1.

Ein besonderes Anwendungsgebiet der Erfindung ist das Verabreichen von, insbesondere an sich für eine Injektion bestimmten, in einer Flüssigkeit in einer Ampulle vorliegenden Wirkstoffen für den menschlichen oder tierischen Körper. Die erfindungsgemäße Sprühvorrichtung ist jedoch auch zum Versprühen anderer Flüssigkeiten allgemeinster Art geeignet.

Die US-A-5 595 326 zeigt eine Vorrichtung zum Versprühen von Pharmazeutika der eingangs angegebenen Art, welche in einer Ampulle enthalten sind, deren Kopf abgebrochen wird. Die Sprüheinrichtung wird dabei auf die Ampulle aufgesteckt. Zur Abdichtung dient eine Art flexible Manschette, welche seitlich am Kopf der Ampulle anliegt, wie in Fig. 3 erkennbar ist. Diese Manschette bildet eine Art Verbindungsadapter für die Ampulle, welcher fest an einer Basis-Sprüheinrichtung angeordnet ist. Eine vom Grundprinzip her ähnliche Ausführungsform ist in den Fig. 6 bis 9 dargestellt. Die Ausführungsform in Fig. 4 und 5 zeigt einen Dichtkopf, welcher unterseitig eine umlaufende Ringnut aufweist, in welche der obere Umfangsrand der Ampulle hineingesteckt wird. - Bei dieser bekannten Sprühvorrichtung besteht die Gefahr, daß sich die Ampulle von der Sprüheinrichtung löst, da die Fixierung einzig und allein durch die Manschette bewerkstelligt wird.

Die US-A-4 526 302 zeigt eine Sprüheinrichtung zum Versprühen beispielsweise von in einer Ampulle enthaltenen Pharmazeutika. Die Sprühvorrichtung weist ein Rohrelement auf, dessen unteres Ende offen ist und einen Längsschlitz aufweist. Im Innern des Rohrelements befindet sich eine Pumpeinrichtung sowie eine umlaufende Dichtlippe. Die Ampulle wird von unten in das Rohrelement eingeschoben, bis sie in Anlage zu der umlaufenden Dichtlippe gelangt. Gehalten wird die Ampulle durch einen nasenartigen Vorsprung des Rohrelements. - Diese Sprüheinrichtung ist aufwendig in der Handhabung, weil die Ampulle von unten her durch eine entsprechende Öffnung in das Rohrelement hineingesteckt werden muß. Zu diesem Zweck muß das Rohrelement im Bereich des Längsschlitzes aufgeweitet werden.

Die US-A-4 361 253 zeigt eine Sprühvorrichtung für den Inhalt von Ampullen, deren Kopf abgebrochen oder abgesägt wird. Die Sprüheinrichtung weist eine Aufnahmeöffnung für die Ampulle auf und ist innenseitig mit einem umlaufenden Dichtring versehen, welcher an der Mantelfläche der Ampulle außenumfänglich anliegt.

Die US-A-5 927 559 zeigt eine Sprüheinrichtung für Pharmazeutika. Das herkömmliche Behältnis wir dabei von unten in eine Art Muffe eingeschoben und liegt dabei mit dem oberen Umfangsrand an einer Dichtung an. Diese Sprüheinrichtung weist einen Filter für den Lufteinlaß auf.

Die WO 95/00195 A zeigt eine Sprüheinrichtung für flüssige Pharmazeutika, welche in einem Behälter enthalten sind. Dieser Behälter ist oberseitig mit einem Gummistopfen verschlossen, welcher von Nadeln durchstoßen wird.

Der Erfindung liegt die **Aufgabe** zugrunde, bei einer Vorrichtung zum Versprühen von Flüssigkeiten der eingangs angegebenen Art die Verbindung zwischen der Sprüheinrichtung und dem Behältnis für die Flüssigkeit zu verbessern.

Die technische **Lösung** ist gekennzeichnet durch die Merkmale im Kennzeichen des Anspruchs 1.

Dadurch ist ein einfach handhabbares und sicheres System zum Versprühen von Flüssigkeiten geschaffen. Die Grundidee besteht darin, ein spezielles Behältnis aus Glas oder Kunststoff für die zu versprühende Flüssigkeit in der Art einer Ampulle mit einer speziellen Sprüheinrichtung zu kombinieren. Die Sprüheinrichtung wird dabei auf das die Flüssigkeit enthaltende Behältnis aufgesetzt, um mittels dieser Sprüheinrichtung die in dem Behältnis befindliche Flüssigkeit zu versprühen. Bei dem Behältnis kann es sich - wie ausgeführt - um eine Ampulle handeln, wie sie üblicherweise für Injektionen verwendet wird. Es ist lediglich erforderlich, den Kopf der Ampulle abzubrechen und auf die dadurch geschaffene Entnahmeöffnung die Sprüheinrichtung zu setzen. Statt der Ampulle kann auch eine entsprechend ausgebildete Flasche vorgesehen sein, in der mehrere Dosiereinheiten enthalten sind. Die Sprüheinrichtung wird dabei luft- und flüssigkeitsdicht direkt auf die Entnahmeöffnung des Behältnisses aufgesetzt. Hierzu dient die Ringdichtung. Bei Ampullen kompensiert dabei diese Ringdichtung entscheidend die teilweise nicht immer exakt regelmäßige Bruchstelle. Auch bei anderen Behältnissen ist jederzeit eine absolute Dichtheit geschaffen, insbesondere wenn die Behältnisse von der Form her leicht variieren. Mittels der Sprüheinrichtung ist eine mechanische Einrichtung zur Verfügung gestellt, um auf einfache Weise die zu applizierende Flüssigkeit auf die gewünschte Schleimhaut zu sprühen. Dadurch ist ein alltagstaugliches und standardisiertes System geschaffen, welches es dem Anwender erlaubt, ohne unzumutbaren Aufwand den Ampulleninhalt zu verabreichen. Damit ist dieses System für viele Injektionsgebiete geeignet. Die mechanische Einrichtung für die mucosale Applikation von in Ampullen befindlichen Arzneimitteln mittels Pumpsprüheinheit besteht aus einer Pumpe, einem Sprühkopf, einem Ampullenkonnektor sowie aus einer Ampulfenfixiereinheit. Wie eingangs bereits erwähnt, kann es sich aber auch um andere Flüssigkeiten handeln, welche sich in dem Behältnis befinden.

Die Sprüheinrichtung ist erfindungsgemäß aus zwei Grundelementen gebildet. Das Basiselement stellt dabei die Basis-Sprüheinrichtung dar, d.h. eine universelle Sprüheinrichtung, welche für die unterschiedlichsten Behältnisse verwendbar ist. Die individuelle Anpassung an das jeweilige Behältnis erfolgt durch ein zweites Element, nämlich dem Verbindungsadapter. Dieser wird auf der Basis-Sprüheinrichtung individuell sowie dicht aufgesetzt. Mittels dieses Verbindungsadapters ist eine einwandfreie Verbindung zu dem jeweiligen Behältnis gegeben. Der Vorteil dieses Systems besteht in der Kostengünstigkeit, weil nur eine einzige Basis-Sprüheinrichtung benötigt wird. Die Verbindungsadapter sind hingegen einfach in der Herstellung. Durch ein entsprechendes Sortiment kann somit jedes beliebige Behältnis adaptiert werden. Bei der Basis-Sprüheinrichtung kann es sich um eine Handelsware handeln, aber auch um eine spezielle Entwicklung. Die Basis-Sprüheinrichtung sowie der Verbindungsadapter können fest miteinander verbunden sein. Im Sinne einer größeren Flexibilität können die beiden Elemente jedoch auch auswechselbar sein, um - wie zuvor beschrieben - Grundpumpsysteme mit unterschiedlichen Dosiervolumina einzusetzen.

Die Grundidee hinsichtlich der Halterung des Behältnisses an der Sprüheinrichtung besteht darin, das Behältnis, insbesondere die Ampulle, mit dem abgebrochenen Kopfende in die Hülse hineinzustecken und anschließend von oben den Verbindungsadapter aufzustecken oder aufzuschrauben, und zwar bis die Ringdichtung in Anlage zur Schulter des Behältnisses gelangt. Dadurch ist eine Halterung für das Behältnis geschaffen, bei welchem das Behältnis sicher und geschützt an der Sprüheinrichtung angeordnet ist. Mit dem aufgesteckten oder aufgeschraubten Verbindungsadapter ist darüber hinaus zusammen mit der Hülse eine kompakte Baueinheit geschaffen. Durch die zylinderförmige Ausbildung des Verbindungsadapters und der korrespondierenden zylinderförmigen Ausbildung der Hülse ist eine relativ große Kontaktfläche und damit sichere Fixierung des Verbindungsadapters innerhalb der Hülse gewährleistet. Insbesondere ist es dadurch möglich, durch entsprechende Längengebungen einerseits der Hülse sowie andererseits des Verbindungsadapters unterschiedlich hohe Behältnisse in ein und demselben System verwenden zu können. Die Ringdichtung am unteren Ende des Verbindungsadapters ist vorzugsweise eine angespritzte Dichtung, so daß der Verbindungsadapter und die Ringdichtung eine einteilige Baueinheit bilden. Das Material der Ringdichtung ist dabei ein anderes Material als das Material des Verbindungsadapters.

Die generellen Vorteile sind wie folgt:
- Maximum an Betätigungssicherheit
- einfach im Gebrauch
- Dosiergenauigkeit
- produktschonend
- Reduktion des Zeitaufwandes und der Materialkosten

Die Weiterbildung gemäß Anspruch 2 beschreibt drei verschiedene Arten der Dichtung. Bei der Variante mit der Schulter liegt die Ringdichtung von oben auf dieser Schulter auf. Bei der Variante mit der äußeren Mantelfläche liegt die Ringdichtung außen am eigentlichen Mantel (also unterhalb der Schulter) des Behältnisses an, während bei der letzten Variante die Ringdichtung in die Entnahmeöffnung eintaucht.

Wie zuvor bereits erwähnt, handelt es sich gemäß der Weiterbildung in Anspruch 3 bei der Sprüheinrichtung vorzugsweise um eine Pump-Sprüheinrichtung. Nach dem Nachuntendrücken des Betätigungsknopfes wird bei der anschließenden selbständigen Nachobenbewegung des Betätigungsknopfes in dem Sprühkopf ein Unterdruck erzeugt, welcher die in dem Behältnis befindliche Flüssigkeit durch das Steigrohr hoch zum Sprühkopf saugt. Während des anschließenden Nachuntendrückens des Betätigungsknopfes wird der Ansaugkanal geschlossen und die im Sprühkopf befindliche, zuvor angesaugte Flüssigkeit durch die Düse hinausgedrückt. Der Vorteil dieses Systems im Vergleich zu einem Pumpsystem unter Verwendung von Überdruck im Behälter besteht darin, daß keine luftdichte Abdichtung des Sprühkopfes zum Behälter notwendig ist. Dadurch ist eine einfache mechanische Einrichtung zur technischen Realisierung der Sprüheinrichtung geschaffen. Außerdem ist durch das Pumpsystem die Dosierung auf einfache Weise reproduzierbar. Als Dosiervolumina können 0,01 ml/Hub bis 0,2 ml/Hub verwendet werden. Durch diesen Einsatz der Pump-Sprüheinrichtung mit einem vorgegebenen Hubvolumen ist es möglich, entsprechend definierte Dosen des entsprechenden Mittels nach Bedarf und Wunsch zu applizieren. Auch kann die Anzahl der Pumphübe eingestellt, d.h. begrenzt werden. Statt der Förderung der Flüssigkeit mittels eines Pumpsystems sind auch andere Fördersysteme denkbar, welche den entsprechenden Zweck erfüllen und die Flüssigkeit dem Sprühkopf zuführen.

Der Vorteil der Weiterbildung gemäß Anspruch 4 besteht in einem Keimschutz.

Grundsätzlich kann das Behältnis auf den Kopf mit der Öffnung nach unten gestellt werden. Dadurch kann auf ein Steigrohr verzichtet werden. Die Weiterbildung gemäß Anspruch 5 schlägt jedoch die Verwendung des vorgenannten Steigrohres vor. Dadurch befindet sich die Sprüheinrichtung oben und kann daher besser bedient werden.

Die Weiterbildung gemäß Anspruch 6 hat den Vorteil, daß das Steigrohr gemäß dem Bestimmungszweck der jeweiligen Ampullenlänge angepaßt werden kann.

Die Weiterbildung gemäß Anspruch 7 hat den Vorteil, daß Verunreinigungen, welche beim oder nach dem Öffnen der Ampulle in die Flüssigkeit gelangen können, ausgefiltert werden.

Die Weiterbildung gemäß Anspruch 8 schlägt verschiedene Arten der Verbindung zwischen der Hülse und dem Verbindungsadapter vor. Bei einer lösbaren Verrastung ist eine Wiederverwendung des Systems möglich, indem nach Gebrauch der Verbindungsadapter wieder von der Hülse entfernt wird. Die lösbare Verbindung kann beispielsweise durch Rastelemente in Form von umfänglichen Rippen erfolgen. Die nicht lösbare Verbindung zwischen der Hülse und dem Verbindungsadapter ist für den Einmalgebrauch bestimmt. Durch eine entsprechende Ausbildung der Verrastelemente kann die Nichtlösbarkeit erreicht werden. Schließlich kann für eine lösbare Verbindung auch eine Art Bajonettverschluß vorgesehen sein.

Eine weitere bevorzugte Weiterbildung schlägt gemäß Anspruch 9 schließlich vor, daß auf der Sprüheinrichtung ein Sprühkopf auswechselbar anordenbar ist. Der Vorteil dieses auswechselbaren Sprühkopfes besteht darin, daß alle gängigen sowie zukünftigen Adapter, wie beispielsweise Rachenadapter mit und ohne Schnabel, Nasenadapter etc. bedarfsweise eingesetzt werden können.

Ein Ausführungsbeispiel einer erfindungsgemäßen Vorrichtung zum Versprühen von Flüssigkeiten, nämlich zum Verabreichen eines in einer Ampulle vorliegenden Medikaments wird nachfolgend anhand der Zeichnungen beschrieben. In diesen zeigt:

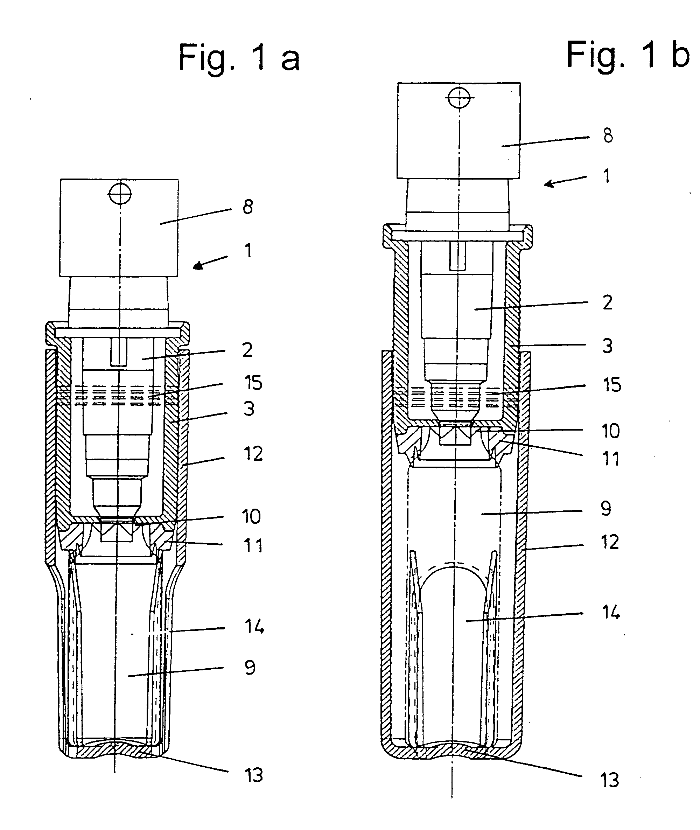

- Fig. 1a: einen Längsschnitt durch die Sprühvorrichtung;
- Fig. 1b: eine Darstellung entsprechend der in Fig. 1 a, jedoch mit einem größeren Behältnis.

Die Sprühvorrichtung der Fig. 1a weist eine Sprüheinrichtung 1 auf. Diese besteht aus einer Basis-Sprüheinrichtung 2 sowie aus einem zylinderförmig ausgebildeten Verbindungsadapter 3. Dieser Verbindungsadapter 3 ist von unten auf die Basis-Sprüheinrichtung 2 aufgesteckt, so daß sich die Basis-Sprüheinrichtung 2 im Verbindungsadapter 3 befindet. Sie wird oben und unten innerhalb dieses Verbindungsadapters 3 in der dargestellten Weise gehalten. Je nach Anwendungsfall können unterschiedliche Formen von Verbindungsadaptern 3 verwendet werden. Im unteren Umfangsbereich weist der Verbindungsadapter 3 eine angespritzte Ringdichtung 11 auf. Die Sprüheinrichtung 1, nämlich deren Basis-Sprüheinrichtung 2 weist unterseitig ein nicht dargestelltes Steigrohr sowie oberseitig einen Sprühkopf 8 auf. Dieser ist austauschbar und somit für den jeweiligen Anwendungsfall anpaßbar.

Weiterhin ist eine Hülse 12 vorgesehen. Diese weist einen Boden 13 sowie im Bodenbereich seitliche Fenster 14 auf. Im oberen Bereich weist die Hülse 12 innumfänglich Rastrippen 15 auf. Der Verbindungsadapter 3 weist zu den Rastrippen 15 korrespondierende Rastelemente auf.

### Die Funktionsweise ist wie folgt:

Das Sprühsystem soll an ein mit einem Medikament gefülltes Behältnis 9 in Form einer Ampulle angeschlossen werden. Hierzu wird zunächst der Glaskopf des Behältnisses 9 abgebrochen.

Anschließend wird das Behältnis 9 in die Hülse 12 von oben hineingesteckt. Danach wird der Verbindungsadapter 3 der Sprüheinrichtung 1 von oben auf die Hülse 12 aufgesteckt und solange nach unten verschoben, bis die Ringdichtung 11 auf der Schulter des Behältnisse 9 dichtend zu liegen kommt. In dieser Position sind die Rastrippen 15 der Hülse 12 mit entsprechenden Rastelementen des Verbindungsadapters 3 verrastet. Durch eine entsprechende Ausbildung dieser Rastrippen 15 können die Hülse 12 und der Verbindungsadapter 3 lösbar oder aber auch nicht lösbar sein.

Durch Betätigen des Pumpsprühkopfes 8 kann die in Behältnis 9 befindliche Flüssigkeit mit dem eintauchenden Steigrohr versprüht werden.

Die Darstellung in Fig. 1b zeigt ein Behältnis 9 mit einem größeren Volumen, so daß das Behältnis 9 im Vergleich zum Behältnis 9 der Ausführungsform in Fig. 1 a höher ist. Auch dieses Behältnis 9 wird in die Hülse 12 hineingesteckt und anschließend der Verbindungsadapter 3 von oben aufgeschoben. In diesem Fall ragt der Verbindungsadapter 3 nicht so weit nach unten wie bei der Ausführungsform in Fig. 1 a, da bereits früher die Schulter des Behältnisses 9 erreicht wird.

Als Behältnis 9 sind nicht nur Ampullen denkbar, sondern jede Art von Behältnis 9. Auch kann die Ringdichtung 11 an anderen Bereichen als die Schulter am Behältnis 9 angreifen, nämlich außen am Mantel oder innen im Bereich der Entnahmeöffnung 10.

### Bezugszeichenliste

- 1: Sprüheinrichtung
- 2: Basis-Sprüheinrichtung
- 3: Verbindungsadapter
- 8: Sprühkopf
- 9: Behältnis
- 10: Entnahmeöffnung
- 11: Ringdichtung
- 12: Hülse
- 13: Boden
- 14: Fenster
- 15: Rastrippen

## Patentansprüche

1. Vorrichtung zum Versprühen von Flüssigkeiten,
wobei die Flüssigkeit in einem im Ursprungszustand einstückig geschlossenen Behältnis (9), insbesondere Ampulle, mit einem abbrechbaren oder absägbaren Kopf zur Schaffung einer Entnahmeöffnung (10) enthalten ist,
die Vorrichtung mit einer auf der Entnahmeöffnung (10) des Behältnisses (9) anordenbaren Sprüheinrichtung (1) aus einer Basis-Sprüheinrichtung (2) und aus einem fest darauf angeordneten Verbindungsadapter (3) für das Behältnis (9),
wobei der Verbindungsadapter (3) eine Ringdichtung (11) aufweist, welche dichtend am Behältnis (9) anliegt,
**dadurch gekennzeichnet,**
**daß** der Verbindungsadapter (3) im wesentlichen zylinderförmig ausgebildet ist und an seinem unteren Umfangsende die Ringdichtung (11) aufweist,
**daß** das Behältnis (9) in einer, am unteren Ende einen Boden (13) aufweisenden Hülse (12) aufgenommen ist und
**daß** die Hülse (12) mit ihrem oberen, offenen Ende auf den Verbindungsadapter (3) aufgesteckt oder aufgeschraubt ist.

2. Vorrichtung nach dem vorhergehenden Anspruch,
**dadurch gekennzeichnet,**
**daß** das Behältnis (9) im Bereich der Entnahmeöffnung (10) eine Schulter aufweist, auf welcher die Ringdichtung (11) aufliegt,
und/oder
**daß** die Ringdichtung (11) außen am Behältnis (9) im Mantelbereich anliegt
und/oder
**daß** die Ringdichtung (11) innen am Behältnis (9) im Bereich der Entnahmeöffnung (10) anliegt.

3. Vorrichtung nach einem der vorhergehenden Ansprüche,
**dadurch gekennzeichnet,**
**daß** die Sprüheinrichtung (1) eine Pump-Sprüheinrichtung ist.

4. Vorrichtung nach einem der vorhergehenden Ansprüche,
**dadurch gekennzeichnet,**
**daß** dem Lufteinlaß der Sprüheinrichtung (1) ein Mikrofilter zugeordnet ist.

5. Vorrichtung nach einem der vorhergehenden Ansprüche,
**dadurch gekennzeichnet,**
**daß** die Sprüheinrichtung (1) ein ins Innere bis etwa zum Boden des Behältnisses (9) reichendes Steigrohr (7) aufweist.

6. Vorrichtung nach Anspruch 5,
**dadurch gekennzeichnet,**
**daß** das Steigrohr (7) auswechselbar ist oder vorgegebene Sollbruchstellen für eine Kürzung aufweist.

7. Vorrichtung nach einem der vorhergehenden Ansprüche,
**dadurch gekennzeichnet,**
**daß** der Sprüheinrichtung (1 ), insbesondere dem Steigrohr (7) ein Mikrofilter für die Flüssigkeit zugeordnet ist.

8. Vorrichtung nach einem der vorhergehenden Ansprüche,
**dadurch gekennzeichnet,**
**daß** die Verbindung zwischen der Hülse (12) und dem Verbindungsadapter (3) lösbar verrastbar, nicht lösbar verrastbar oder durch eine Art Bajonettverschluß gebildet ist.

9. Vorrichtung nach einem der vorhergehenden Ansprüche,
**dadurch gekennzeichnet,**
**daß** auf der Sprüheinrichtung (1) ein Sprühkopf (8) auswechselbar anordenbar ist.

## Claims

1. Device for spraying liquids, the liquid being contained in a container (9) closed in one piece in the original state, in particular an ampoule, with a break-off or saw-off head for creating a removal aperture (10), the device comprising a spray apparatus (1), which can be arranged on the removal aperture (10) of the container (9), composed of a basic spray apparatus (2) and a connection adapter (3) rigidly arranged thereon for the container (9), the connection adapter (3) having an annular seal (11) which rests in a sealing manner on the container (9), **characterised in that** the connection adapter (3) is substantially cylindrical in design and on its lower peripheral end has the annular seal (11), **in that** the container (9) is received in a sleeve (12) having a base (13) at the lower end, and **in that** the sleeve (12) is placed or screwed by its upper, open end onto the connection adapter (3).

2. Device according to the preceding claim, **characterised in that** the container (9), in the region of the removal aperture (10), has a shoulder, on which the annular seal (11) rests, and/or **in that** the annular seal (11) rests on the outside of the container (9) in the lateral surface region, and/or **in that** the annular seal (11) rests on the inside of the container (9) in the region of the removal aperture (10).

3. Device according to any one of the preceding claims, **characterised in that** the spray apparatus (1) is a pump spray apparatus.

4. Device according to any one of the preceding claims, **characterised in that** a microfilter is associated with the air inlet of the spray apparatus (1).

5. Device according to any one of the preceding claims, **characterised in that** the spray apparatus (1) has a riser pipe (7) extending into the interior up to about the base of the container (9).

6. Device according to claim 5, **characterised in that** the riser pipe (7) is replaceable or has predetermined breaking points for shortening.

7. Device according to any one of the preceding claims, **characterised in that** a microfilter for the liquid is associated with the spray apparatus (1), in particular the riser pipe (7).

8. Device according to any one of the preceding claims, **characterised in that** the connection between the sleeve (12) and the connection adapter (3) is detachably lockable, non-detachably lockable, or is formed by a type of bayonet catch.

9. Device according to any one of the preceding claims, **characterised in that** a spray head (8) can be replaceably arranged on the spray apparatus (1).

## Revendications

1. Dispositif pour pulvériser des liquides, sachant que le liquide est contenu dans un récipient (9) clos d'un seul tenant à l'origine, notamment dans une ampoule, avec une extrémité sécable ou sciable pour créer une ouverture de prélèvement (10), que le dispositif comprend ou est pourvu d'un pulvérisateur (1) pouvant être disposé sur l'ouverture de prélèvement (10) du récipient (9), lequel pulvérisateur est lui-même constitué d'un pulvérisateur de base (2) et d'un adaptateur de liaison (3) pour le récipient (9) monté sur le pulvérisateur de base, sachant que l'adaptateur de liaison (3) présente une bague d'étanchéité (11) qui est appliquée contre le récipient (9) de manière à réaliser l'étanchéité, **caractérisé en ce que** l'adaptateur de liaison (3) est réalisé essentiellement en forme de cylindre et présente la bague d'étanchéité (11) à son extrémité inférieure, **en ce que** le récipient (9) est logé dans une douille (12) dotée d'un fond (13) à son extrémité inférieure, et **en ce que** la douille (12) est enfoncée ou vissée par son extrémité supérieure ouverte sur l'adaptateur de liaison (3).

2. Dispositif selon la revendication précédente, **caractérisé en ce que** le récipient (9) présente dans la zone de l'ouverture de prélèvement (10) un épaulement sur lequel repose la bague d'étanchéité (11), et/ou **en ce que** la bague d'étanchéité (11) est appliquée contre l'extérieur du récipient (9), dans la zone de l'enveloppe latérale, et/ou **en ce que** la bague d'étanchéité (11) est appliquée contre l'intérieur du récipient (9), dans la zone de l'ouverture de prélèvement (10).

3. Dispositif selon l'une des revendications précédentes, **caractérisé en ce que** le pulvérisateur (1) est un pulvérisateur à pompe.

4. Dispositif selon l'une des revendications précédentes, **caractérisé en ce qu'**un microfiltre est associé à l'entrée d'air du pulvérisateur (1).

5. Dispositif selon l'une des revendications précédentes, **caractérisé en ce que** le pulvérisateur (1) présente un tuyau de montée (7) rentrant à l'intérieur du récipient (9) et s'étendant presque jusqu'au fond de celui-ci.

6. Dispositif selon la revendication 5, **caractérisé en ce que** le tuyau de montée (7) est remplaçable ou présente des points de rupture prédéterminés pour le raccourcir.

7. Dispositif selon l'une des revendications précédentes, **caractérisé en ce qu'**un microfiltre pour le liquide est associé au pulvérisateur (1), notamment au tuyau de montée (7).

8. Dispositif selon l'une des revendications précédentes, **caractérisé en ce que** la liaison entre la douille (12) et l'adaptateur de liaison (3) peut être enclenchée de façon amovible, de façon non amovible ou est constituée par une sorte de fermeture à baïonnette.

9. Dispositif selon l'une des revendications précédentes, **caractérisé en ce qu'**une tête de pulvérisation (8) peut être montée de façon interchangeable sur le pulvérisateur (1).
